# EUROPEAN PATENT APPLICATION

(11) **EP 4 477 180 A1**
(43) Date of publication of application: **18.12.2024**
(21) Application number: 23179738.2
(22) Date of filing: 16.06.2023
(51) Int. Cl.: A61B 90/00, A61B 34/10

(54) **SYSTEM AND METHODS FOR DISTINGUISHING ANGULAR SECTIONS OF VIEWING DIRECTIONS**

(71) Applicant: metamorphosis GmbH, 33184 Altenbeken (DE)
(72) Inventor: Blau, Arno, 72336 Balingen (DE); Lamm, Artur, 33102 Paderborn (DE)
(74) Representative: LKGLOBAL Lorenz & Kopf Patentanwalt Attorney at Law PartG mbB

(57) **Abstract**

A system for differentiating angular section when imaging an object is provided. The system may comprise an imaging device (such as a C-arm) configured to generate projection images with a projection direction being defined for at least one point of the object. The imaging device may be movable so that the projection direction for the at least one point of the object rotates about the at least one point, wherein the system may be configured to image the object together with surrounding structure and to identify a first angular section in which at least two positions or two orientations of the object relative to the surrounding structure result in the same projection image, when imaging the object along the projection direction through the at least one point of the object.

## Description

### FIELD OF INVENTION

The invention relates to the field of computer-assisted surgery. The systems and methods described herein can be applied to assist in performing steps in surgical procedures. In particular, the systems and methods aim in identifying viewing directions which might have a plurality of relations between objects in 3D space resulting in the same projection image. The methods can be implemented as computer program product which can be executed on a processing unit of a system.

### BACKGROUND OF THE INVENTION

Conventional minimally invasive spinal surgery is typically done with intraoperative X-ray imaging control. The challenge is that these X-ray images are projection images, i.e. are two-dimensional only, and in order to imagine 3D information there is need for repeatedly changing between lateral ML and frontal AP position of a C-arm. On the one hand, this takes time which is especially in an operation room environment very expensive and raises the probability of infection and, on the other hand, it requires a lot of x-ray images, e.g. for a single drilling of pedicle hole, which ends up in a significant radiation exposure of patient and operating room staff. Therefore, surgeons often just acquire frontal X-ray images at least until the drilling is past a certain amount and then also check the position in a lateral image.

But this procedure has the risk, depending on C-arm position (imaging direction) that the start point for the drilling onto a bone has ambiguities in the 2D projection X-ray image. Thus, it can happen that the surgeon believes that an instrument like a drill bit is on an intended 3D position but in reality it is several millimeters away therefrom.

Computer-assisted surgery (CAS) is a rapidly advancing field that aims to enhance surgical procedures through the use of computer technology. While CAS offers numerous benefits, there are also several challenges and problems associated with its implementation.

Surgeons and surgical teams need to undergo training to become proficient in using computer-assisted surgery systems. Learning the technical aspects, mastering the user interfaces, and understanding the integration of CAS into surgical workflows can take time, potentially affecting the efficiency and speed of surgical procedures during the initial learning period.

While computer-assisted surgery systems aim to improve surgical precision, there can be challenges regarding the accuracy and reliability of the technology. Factors such as tracking errors, image registration inaccuracies, and system calibration issues can affect the overall precision of the surgical guidance provided by CAS systems.

### SUMMARY OF THE INVENTION

At least the one or the other of the mentioned problems are mitigated or solved by the subject matter according to each of the independent claims. Further embodiments are described in the respective dependent claims. A computer-assisted surgery system is suggested which may involve image-guided navigation, robotic systems, and surgical simulation. The integration of these technologies into surgical workflows is proposed herewith and a training for surgeons and operating room staff is facilitated.

In general, a system for differentiating angular section, when imaging an object, comprises an imaging device (such as a C-arm) configured to generate projection images with a projection direction being defined for at least one point of the object. The imaging device is movable so that the projection direction for the at least one point of the object rotates about the at least one point, wherein the system is configured to image the object together with surrounding structure and to identify a first angular section in which at least two positions or two orientations of the object relative to the surrounding structure result in the same projection image, when imaging the object along the projection direction through the at least one point of the object.

In other words, the imaging device may be capable of generating projection images from different angles around an object by moving and rotating the projection direction. The system may be designed to capture the object along with its surrounding structure and identify a specific angular section where multiple positions or orientations of the object relative to the surrounding structure result in the same projection image. By identifying the angular section where similar projection images occur, the system can help differentiate between different views or orientations of the object.

The point which can be considered as center of rotation may, for example, be a point on a surface of a bone. In the context of this disclosure, it is to be understood that the point may be relevant in the context of a surgical procedure. The point may be a specific point but not any point on a line representing a projection beam. The mentioned point may be on a surface of a bone and may be defined by an instrument like a drill which is placed in contact with the bone surface before drilling. In consequence, it will be understood that it depends on the direction from which a surgeon approaches an anatomic structure which influences which point is relevant. Considering a projection beam extending through the anatomic structure, e.g., through a bone, will typically result in at least two points along the projection beam, one at the front of the bone and another one at the back side of the bone. For the next steps of a surgical procedure, the point at the front is typically relevant. In consequence, only those points or structures may be taken into account when assessing an actual position of a point, which can be identified as lying in the vicinity of a tip of an instrument, e.g. lie within the range of 20 mm, or within the range of 10 mm, or particularly within the range of 3 mm away from the tip of the instrument. According to an example, the range may be between 2 mm and 10 mm, or alternatively between 2 mm and 20 mm.

The identification of the first angular section may be based on a transfer of the projection direction through the at least one point of the object as a line into a 3D data set representing the surrounding structure. Throughout this disclosure, the term "projection direction" means the 3D angle which describes the direction in which a chosen X-ray beam (e.g., a central X-ray beam) passes through a chosen point of an object. A central beam of a projection imaging device in the example of a C-arm is the beam between the focal point and the center of the projection plane (in other words, it is the beam between the focal point and the center of the projection image). In consequence, the "projection direction" may also be called "imaging direction".

According to an example, the object may be a drill bit and the surrounding structure may be a vertebra of a spine. The two positions of the object may, thus, be contact points of the tip of the drill bit on the bone surface of a first process of the vertebra and on the bone surface of a second process of the vertebra, wherein the first process of the vertebra lies in front of the second process of the vertebra, when considering the two processes along the line. Alternatively, the two positions of the object may be at a distance from each other on a bone surface which lies along the path of an X-ray beam passing through the point of contact between object and bone surface.

In other words, the system utilizes a transfer of the projection direction through the at least one point of the object, e.g. of a drill tip, represented as a line, into a 3D data set that represents the surrounding structure. This approach allows for the identification of the first angular section where the projection images remain the same when imaging the object along the projection direction. In the given example, the system identifies the first angular section by considering the two positions of, e.g., the drill bit, which correspond to contact points of the drill bit's tip on the bone surface of the vertebra.

By identifying this first angular section, the system can determine a range of positions or orientations of the drill bit relative to the vertebra that would produce the same projection image. This information can be valuable for surgical planning or guidance, as it helps differentiate between different parts of the vertebra and provides insights into the relative positions of the drill bit and the bone surfaces.

That example shows that the point, which is relevant, is a point on the bone surface, e.g., at one of the processes of a vertebra, identified by the side and direction from which a surgeon approaches that process during the surgical procedure. In particular, a second point can be identified on the vertebra which can be approached from the same side and direction. Assuming that a surgeon has approached more or less the second process of the vertebra although the first process was intended, misplacement of a screw later inserted into the drilled bore may be the result.

According to another example, the object may be an implant having a transverse bore and the two orientations of the object have different angles of rotation of the implant about an axis of the implant and relative to the imaging device.

When imaging the implant, the system captures projection images from different orientations by rotating the implant about its axis, or by rotating the imaging device. By comparing these projection images, the system can identify angular sections where the images remain the same or exhibit minimal variation.

For instance, if the implant is rotated by a certain angle around its axis, it may result in a distinct projection image. However, if the implant is rotated by a different angle, the projection image may be identical or exhibit little variation compared to the previous orientation. This indicates that the implant has entered a different angular section, where multiple orientations result in identical or similar projection images.

By identifying these angular sections, the system provides valuable information for analyzing and understanding the implant's positioning, alignment, and relationship to the surrounding structures.

In the context of the system and method for differentiating angular sections as disclosed herein, the term 'same' is defined to encompass cases where the projection images obtained from different positions or orientations of the object relative to the surrounding structure exhibit minimal discernible variation. Two projection images are considered 'same' if they meet one or more of the following criteria:
- Pixel intensity similarity: The pixel intensity values of the corresponding pixels in the images differ by a specified threshold or percentage. A small difference within the defined threshold is permissible.
- Structural alignment: The structural features, such as edges, contours, or salient landmarks, in the images align within an acceptable tolerance. This alignment can be measured using geometric or statistical measures of similarity.
- Overlapping regions: A specific percentage or statistical measure of overlap between the images is above a predefined threshold. This indicates a substantial similarity in the areas of the object captured by the images.

The defined criteria aim to capture cases where the projection images are nearly identical or exhibit minor variations due to noise or inherent limitations in the imaging process. By considering such similarities, the method can effectively identify the first angular section where multiple positions or orientations of the object produce projection images that are deemed 'same' according to the established criteria.

It is important to note that the specific values, thresholds, and measures mentioned in the definition should be tailored to a specific application and imaging system, considering the level of tolerance and precision required in the context of a medical application. For example, the specific values, thresholds and measures will differ when comparing a video imaging device and an X-ray imaging device.

Independent from the examples, the identification of the first angular section may be based on 3D data representing the object and/or the surrounding structure. That is, angular section may be identified during the planning, which sections would result in similar projection images based on two or more positions or orientations of an object. 3D data may be generated by a CT scan.

The projection direction may alternatively or additionally be determined based on one projection image generated by the imaging device. The imaging device may be an X-ray imaging device.

According to an embodiment, the system may further be configured to identify a second angular section in which only one position and orientation of the object result in the projection image, when imaging the object along the projection direction through the at least one point. As already mentioned above, a point of contact on the surface of a bone may be such at least one point, wherein the side from which the bone is approached is kept in mind. It may be understood that finally a plurality of angular sections may be determined, one or more sections with an unambiguous position and orientation of the object and one or more sections in which the object may have more than one possible position or orientation leading to one projection image. Those sections may, in particular, be defined in 3D space, meaning based on different rotation axes for the angular sections. When considering a C-arm based X-ray imaging system, for example the propeller axis and the C-arm rotation may define the angular sections in 3D space.

A method of differentiating angular sections may comprise the step of imaging an object together with surrounding structure by means of an imaging device generating a projection image with a projection direction being defined for at least one point of an imaged object, wherein the imaging device is movable so that the projection direction for the at least one point of the object rotates about the at least one point. The method may further comprise the steps of identifying a first angular section in which at least two positions or two orientations of the object relative to the surrounding structure result in the same projection image, when imaging the object along the projection direction through the at least one point of the object.

It may be noted that an advantage of the described method of differentiating angular sections may be seen in an improved accuracy in assessing the object's position and orientation. By identifying the first angular section where similar projection images occur, the method allows for a more precise understanding of the object's alignment or relationship to the surrounding structures.

Another advantage may be an enhanced efficiency in data analysis. By focusing on the first angular section where multiple positions or orientations result in the same projection image, the method reduces the need for exhaustive analysis of every possible position or orientation. This can save time and computational resources, streamlining the interpretation of imaging data.

Additionally, the method offers a practical approach for characterizing different angular sections without the need for complex algorithms or specialized equipment. By leveraging the movement and rotation capabilities of the imaging device, the method provides a straightforward means to differentiate angular sections based on projection images obtained from different angles.

Overall, the method's advantage lies in its ability to identify angular sections where similar projection images occur, enabling improved accuracy, efficiency, and simplicity in analyzing the object's position, orientation, and relationship to the surrounding structures.

As discussed above, the object may be a drill bit with a tip and the surrounding structure may be a vertebra of a spine. In that case, the identification of the first angular section may include the step of transferring of the projection direction through the at least one point of the object as a line into a 3D data set representing the surrounding structure, wherein the two positions of the object are a first contact points of the tip of the drill bit on the bone surface of a first process of the vertebra and a second contact point of the tip of the drill bit on the bone surface of a second process of the vertebra, wherein the first process of the vertebra lies in front of the second process of the vertebra, when considering the two processes along the line.

Alternatively, the object may be an implant having a transverse bore. Here, the two orientations of the object relative to the surrounding structure which result in the same projection image, may have different angles of rotation of the implant about an axis of the implant and relative to the projection direction of the imaging device.

Practically, it may be determined whether there might be a plurality of possible points which would result in similar projection images, by simulating the points at a beam path in the 3D data and determining the number of edges, e.g. at bone surfaces. Thus, during the planning of, e.g., pedicle holes, it may be determined if there is a viewing direction where both the entry point is free of ambiguities and the angle for the angle instruction is acceptable. It may be noted that the required slope angle for the drilling instruction can be tight because one should always approach from a larger angle which reduces the probability of hand in the beam cone. To ensure this, augmented reality tracking may determine the approximate position of the drill bit to the trajectory and an instruction may be provided that the angle of the drill bit should still be deliberately larger than later required for drilling.

### BREIF DESCRIPTION OF THE DRAWINGS

Fig. 1 shows a C-arm based imaging device with its rotation and translation axes.
Fig. 2 illustrates an example of X-ray projection images of a spine with indications of a drill trajectory and a viewing direction.
Fig. 3 is an X-ray image illustrating the example of Fig. 2 along the viewing direction indicated in Fig. 2.
Fig. 4 illustrates an example of a bone nail with a transverse bore as projections from different imaging directions.
Fig. 5 is a flow chart illustrating a method in accordance with the disclosure.

Throughout the drawings, the same characters, unless otherwise stated, are used to denote like features, elements, components, or portions of the illustrated embodiments. Moreover, while the present disclosure will now be described in detail with reference to the figures, it is done so in connection with the illustrative embodiments and is not limited by the particular embodiments illustrated in the figures.

### DETAILED DESCRIPTION OF THE EMBODIMENTS

In the context of this disclosure, "angular sections" are intended to be identified. Considering a C-arm based x-ray imaging device, the x-ray source together with the x-ray detector may be rotated about an axis of the C-arm. In other words, the imaging device may describe a circle, wherein angular sections can be understood as sections of the circle in a circumferential direction.

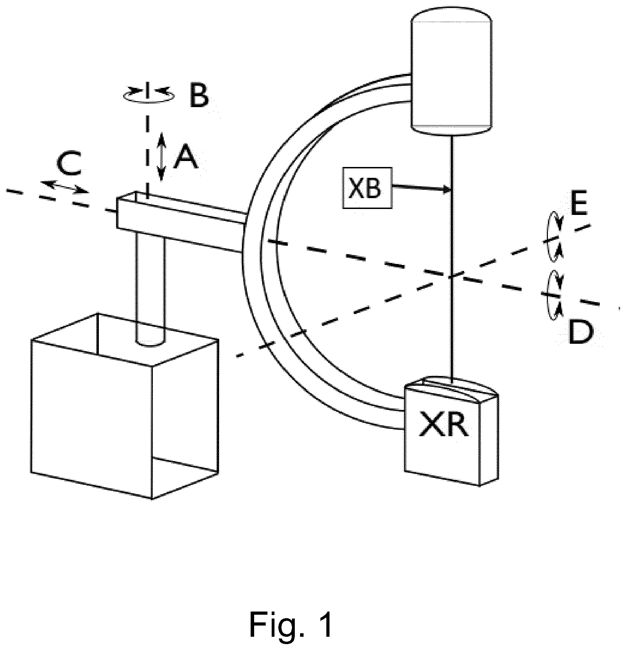

For the definition of a C-arm's rotation and translation axes, it is referred to Fig. 1. In this figure, the X-ray source is denoted by XR, the rotation axis denoted by the letter B is called the vertical axis, the rotation axis denoted by the letter D is called the propeller axis, and the rotation axis denoted by the letter E will be called the C-axis. It is noted that for some C-arm models, the axis E may be closer to axis B. The intersection between axis D and the central X-ray beam (labeled with XB) is called the center of the C-arm's "C". The C-arm may be moved up and down along the direction indicated by the letter A. The C-arm may also be moved along the direction indicated by the letter C. The distance of the vertical axis from the center of the C-arm's "C" may differ between C-arms. It is noted that it may also be possible to use a G-arm instead of a C-arm, with comparable possibilities of movements.

In the following description of specific examples, the imaging device may be positioned so that the center of a rotational movement may correspond to a point of interest within the anatomy of a patient.

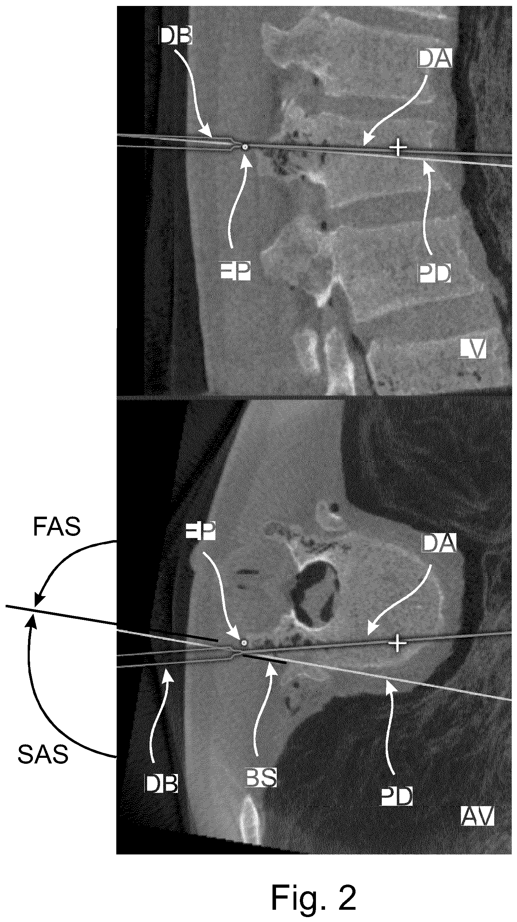

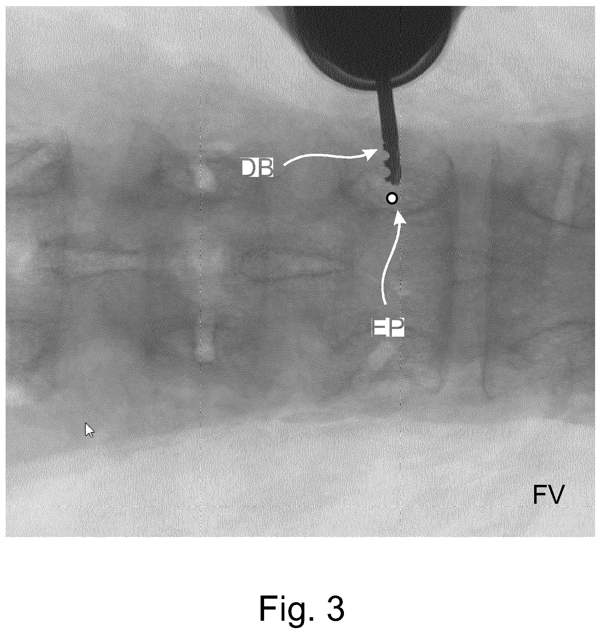

The example shown in Figs 2 and 3 illustrate a situation in which a vertebra of a spine shall be treated, and a drill is to be positioned on the surface of the vertebra before drilling into the bone of the vertebra. Fig. 2 illustrates slices out of a 3D data set of the spine, while Fig. 3 shows an intra-operative X-ray projection image. The upper half of Fig. 2 shows a lateral view LV of the spine and the lower half of Fig. 2 shows an axial view AV of one of the vertebrae. In the upper half of Fig. 2, an outer contour of a drill bit DB is shown with the tip of the drill bit being in the vicinity of an entry point EP on the surface of one of the vertebrae. In the extension of the drill bit DB, a drill axis DA has been inserted into the figure illustrating a path along which the drill bit would penetrate the vertebra when drilling. Furthermore, a line PD in Fig. 2 indicates a projection direction, wherein the image shown in Fig. 3 is generated based on that projection direction.

Fig. 3 is a frontal view FV of the spine shown in Fig. 2. In Fig. 3, a drilling machine with the drill bit DB is at least partially visible. Further, the entry point EP is illustrated in Fig. 3. It is noted that the entry point EP may be identified by a system based on a processed 3D data set of the spine during planning of the surgical intervention, for example on the basis of the 3D data set as in Fig. 2. Thus, the entry point as visualized in Figs 2 and 3 may be an intended entry point.

For the situation resulting in an image as shown in Fig. 3, it may be assumed that a surgeon can feel that the tip of the drill bit DB is in contact with the bone of the vertebra and the surgeon can see in the image that the tip of the drill bit is at least almost at the intended entry point EP as planned. However, when illustrating the projection direction of Fig. 3 as line PD into the 3D date set of Fig. 2, it may be recognized that a radiation beam of the projection image seems to be more or less parallel to a section of the surface of the process of the vertebra at BS. While the line illustrating the projection direction PD is white in Fig. 2, the section of a bone surface BS is drawn as black line. In consequence, the tip of the drill bit DB may also be at another point along the section of the bone surface BS so that the tip is visible in a projection image in the same way as in Fig. 3.

When illustrating the line PD into the 3D data set, the surgeon may recognize the possibility of misplacement without further information from the system. It may, however, be seen as a gist of the disclosure that such possible misplacements are identified by the system and that a surgeon or user of the system may receive an alert or at least an indication that the instrument, e.g., the drill bit in Figs 2 and 3, may be positioned at another location as it seems.

Viewing or projection directions which may result in such multiple possible locations based on a projection direction can be identified in the angular section which is indicated in Fig. 2 as first angular section FAS. Based on the specific projection direction PD, the drill bit may slip down along the surface section BS. Alternatively, the drill bit may erroneously be positioned on the lateral process instead of the posterior process (as in Fig. 2), when assuming another projection direction somewhere in the first angular section FAS.

It is noted that the system which is configured to process the 3D data set as well as the data of the intra-operative X-ray image, may identify the first angular section FAS by registering the projection image and the 3D data set so as to determine the line PD, and may assess whether the voxels along the line PD represent edges or points of a bone surface. As soon as more than two of those voxels are identified as being part of a bone surface, the line may allow for more than one position of the drill bit on the bone surface.

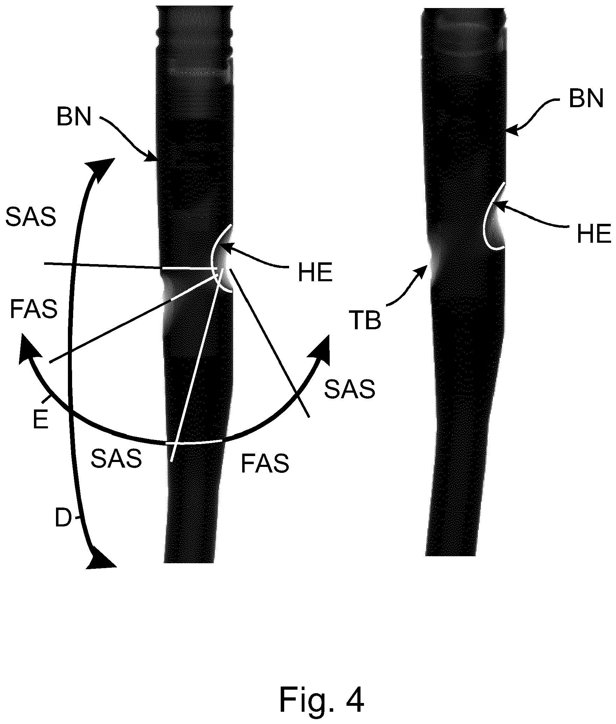

Another example where it is not always possible to determine an actual position and orientation of an object relative to the surrounding structures, is a projection of a bone nail, as illustrated in Fig. 4. Fig. 4 shows on the right side a projection of a bone nail with a first projection direction and on the left side a projection of the bone nail with a second but different projection direction. The bone nail BN in the example comprises a transverse bore TB which extends with an inclined angle through the nail.

When trying to determine the bore axis of the transverse bore TB, e.g., for inserting a bone screw, the hole edge HE may be identified. However, it seems not always possible to decide whether an edge visible in a projection image at the transverse bore TB represents an edge which is nearer or further away from the viewer. Such an edge is schematically indicated in Fig. 4 by a curved white line. Assuming that the actual edge is nearer to the viewer would result in a first angle of rotation of the nail about its longitudinal axis, while assuming that the actual edge is further away would result in a second angle of rotation of the nail about its longitudinal axis. In consequence, a first angular section FAS is drawn into Fig. 4, with viewing or projection directions being in that first angular section allowing for a misinterpretation of the actual orientation of the bone nail.

The projection image of the nail on the left side in Fig. 4 is generated on the basis of a projection direction which is more or less perpendicular to the longitudinal axis of the nail. Further shown in Fig. 4 are arrows D and E which represent possible rotational movements of an imaging device relative to the bone nail BN. For the rotational movements D and E, it is referred to Fig. 1 showing these movements D and E about axes of the imaging device.

In the depict example, the imaging device has been moved both by rotation in the direction of arrow E and by rotation in the direction of arrow D. The projection of the bone nail as on the right side of Fig. 4 results from these rotational movements. As can be recognized, the shape of the projection of the edge HE at the through bore TB has different areas with different appearance of the edge. On the basis of such a projection of the hole edge HE, it is possible to determine the 3D orientation of the bone nail.

That is, a surgeon may by taught to recognize that the image on the left side might cause misinterpretation of the actual orientation of the nail, while the image on the right side can be interpreted more accurate. Alternatively or additionally, the imaging system may be configured to identify projection directions which may be interpreted in a plurality of ways, i.e. may be configured to identify a first angular section. When knowing the imaging direction of a first angular section, imaging directions of second angular sections may be indicated by the system and the user may be provided with a hint in which direction and to which amount the imaging device should be moved so as to be in the second angular section when generating the next image.

The system according to the disclosure may have determined based on the projection direction of the projection on the left side of Fig. 4 that an orientation of an axis of the transverse bore TB may not be determined unambiguously, the system may suggest rotating the imaging device along the arrow D and/or along the arrow E. After such a rotation, the projection as on the right side of Fig. 4 may be generated. Due to a different appearance of the hole edge HE, it is possible for the system to determine the orientation of the axis of the transverse bore TB.

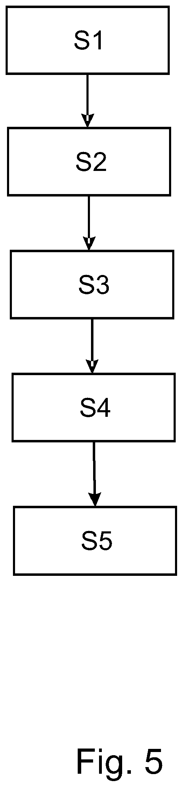

Such steps of a method are also described with reference to the workflow of Fig. 5.

In a first step S 1, an object may be imaged together with surrounding structure by means of an imaging device generating a projection image with a projection direction being defined for at least one point of the imaged object. The imaging device may be movable so that the projection direction for the at least one point of the object may rotate about the at least one point.

According to step S2, a first angular section is identified in which at least two positions or two orientations of the object relative to the surrounding structure result in the same projection image, when imaging the object along the projection direction through the at least one point of the object.

In a case in which the object is a drill with a tip and the surrounding structure is a vertebra of a spine, the projection direction through the at least one point of the object may be transferred as a line into a 3D data set representing the surrounding structure in step S3 so as to identify whether the projection direction lies within the first angular section. As already discussed above, two positions of the object, which result in more or less the same projection, are a first contact point of the tip of the drill on the bone surface of the vertebra and a second contact point of the tip of the drill on the bone surface of the vertebra, with the first contact point lying at a distance to the second contact point.

Alternatively, the object may be an implant having a transverse bore. In that case, a plurality of orientations of the object may be identified in step S3, having different angles of rotation of the implant about an axis of the implant and relative to the projection direction of the imaging device, but resulting in a projection which may be misinterpreted.

According to step S4, a second angular section is identified in which only one position and orientation of the object relative to the surrounding structure results from the projection image, when imaging the object along the projection direction through the at least one point.

Finally, suggestion may be provided in step S5 to a user to change the projection direction so as to be in the second angular section.

With respect to the method steps, it is noted that steps of methods described herein, and in particular of methods described in connection with a workflow, e.g., as visualized in the figure, are major steps, wherein these major steps might be differentiated or divided into several sub-steps. Furthermore, additional sub-steps might be between these major steps. It will also be understood that only part of the whole method may constitute the invention, i.e. steps may be omitted or summarized.

It is furthermore contemplated that the method may be implemented as computer program product to be executed on a processing unit of a system, thus allowing to automatically process X-ray images and identify first angular section for viewing direction for objects of interest.

While embodiments have been illustrated and described in detail in the drawings and aforegoing description, such illustrations and descriptions are to be considered illustrative or exemplary and not restrictive, and the invention is not limited to the disclosed embodiments.

Other variations to the disclosed embodiments can be understood and effected by those skilled in the art in practising the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims.

The mere fact that certain measures are recited in mutually different dependent claims does not indicate that a combination of these measures can not be used to advantage.

## Claims

1. A system for differentiating angular section, when imaging an object, the device comprising an imaging device configured to generate projection images with a projection direction being defined for at least one point of the object, wherein the imaging device is movable so that the projection direction for the at least one point of the object rotates about the at least one point, the system being configured to image the object together with surrounding structure, and to identify a first angular section in which at least two positions or two orientations of the object relative to the surrounding structure result in the same projection image, when imaging the object along the projection direction through the at least one point of the object.

2. The system of claim 1, wherein the identification of the first angular section is based on a transfer of the projection direction through the at least one point of the object as a line into a 3D data set representing the surrounding structure.

3. The system of claim 2, wherein the object is a drill and the surrounding structure is a vertebra of a spine, and wherein the two positions of the object are contact points of the tip of the drill on the bone surface of the vertebra.

4. The system of claim 1, wherein the object is an implant having a transverse bore and wherein the two orientations of the object have different angles of rotation of the imaging device about an axis of the imaging device and relative to the implant.

5. The system of claim 1, wherein the identification of the first angular section is based on 3D data representing the object and/or the surrounding structure.

6. The system of claim 5, wherein the 3D data is generated by a CT scan.

7. The system of any one of claim 1 to 6, wherein the projection direction is determined based on one projection image generated by the imaging device.

8. The system of any one of claims 1 to 7, wherein the imaging device is an X-ray imaging device.

9. The system of any one of claims 1 to 8, wherein the system is further configured to identify a second angular section in which only one position and orientation of the object result in the projection image, when imaging the object along the projection direction through the at least one point.

10. A method of differentiating angular section, the method comprising the step of imaging an object together with surrounding structure by means of an imaging device generating a projection image with a projection direction being defined for at least one point of an imaged object, wherein the imaging device is movable so that the projection direction for the at least one point of the object rotates about the at least one point, the method further comprising the steps of identifying a first angular section in which at least two positions or two orientations of the object relative to the surrounding structure result in the same projection image, when imaging the object along the projection direction through the at least one point of the object.

11. The method of claim 10, wherein the object is a drill with a tip and the surrounding structure is a vertebra of a spine and wherein the identification of the first angular section includes the step of transferring of the projection direction through the at least one point of the object as a line into a 3D data set representing the surrounding structure, wherein the two positions of the object are a first contact point of the tip of the drill on the bone surface of the vertebra and a second contact point of the tip of the drill on the bone surface of the vertebra, wherein the first contact point lies at a distance to the second contact point, when considering the two contact points along the line.

12. The method of claim 10, wherein the object is an implant having a transverse bore and wherein the two orientations of the object have different angles of rotation of the implant about an axis of the implant and relative to the projection direction of the imaging device.

13. The method of any one of claims 10 to 12, wherein the method further comprises the step of identifying a second angular section in which only one position and orientation of the object relative to the surrounding structure results from the projection image, when imaging the object along the projection direction through the at least one point, and providing suggestion to a user to change the projection direction so as to be in the second angular section.
